# EUROPEAN PATENT SPECIFICATION

(11) **EP 0 444 659 B1**
(45) Date of publication and mention of the grant of the patent: **02.10.1996**
(21) Application number: 91102996.5
(22) Date of filing: 28.02.1991
(51) Int. Cl.: A61K 31/445, A61K 47/44

(54) **A pharmaceutical solution comprising derivatives of FK506**
Arzneimittellösung enhaltend Derivate von FK506
Solution pharmaceutique contenant derivées de FK506

(30) Priority: 01.03.1990 JP 51110/90
(43) Date of publication of application: 04.09.1991
(73) Proprietor: FUJISAWA PHARMACEUTICAL CO., LTD., Osaka-shi Osaka 541 (JP)
(72) Inventor: Nakanishi, Shigeo, Neyagawa-shi, Osaka 572 (JP); Yamanaka, Iwao, Osaka-shi, Osaka 547 (JP)
(74) Representative: Türk, Gille, Hrabal, Leifert

(56) References cited:
- EP-A- 0 240 773
- EP-A- 0 315 978

## Description

The present invention relates to a pharmaceutical solution which contains the compound (I) or a pharmaceutically acceptable salt thereof described later that is known as showing an immunosuppressive activity, and to a process for preparing such solution.

In detail, the present invention relates to a non-aqueous pharmaceutical solution of compound (I) which shows long term storage stability and which may be diluted with such as physiological saline, glucose solution for injection, water, and fruit juice without occurence of any precipitation of the compound (I) and which therefore can be applied for various forms of medicine such as intravenous injection, or oral administrating liquidous medicine.

The compound (I) used in the present invention has the general formula 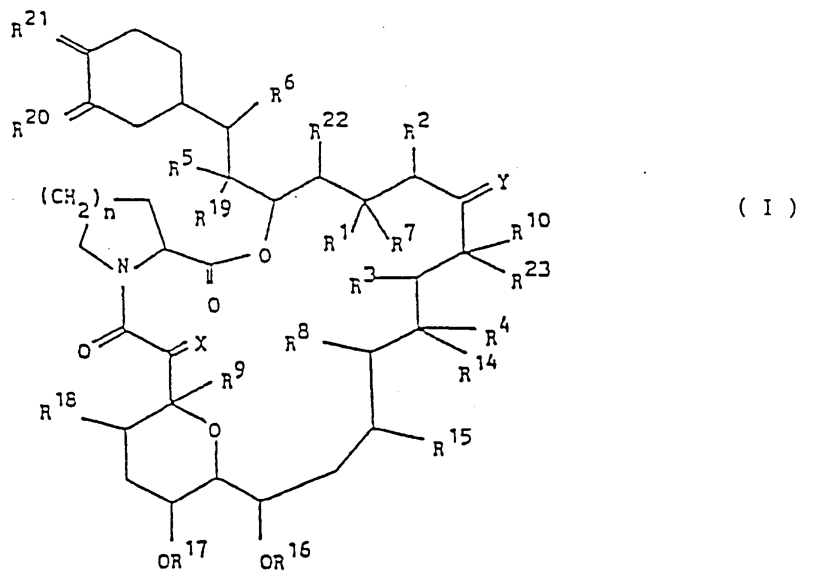 wherein each vicinal pair of substituents [R¹ and R²], [R³ and R⁴], [R⁵ and R⁶] independently
a) represent two vicinal hydrogen atoms, or
b) form a second bond between the vicinal carbon atoms to which they are attached;
in addition to its significance above, R² may represent a C₁-C₆-alkyl group;
- R⁷: represents H, OH, protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy, or O-(C₁-C₆)alkyl,
- R⁸ and R⁹: independently represent H or OH;
- R¹⁰: represents H, C₁-C₆-alkyl, C₁-C₆-alkyl substituted by one or more hydroxyl groups, C₂-C₆-alkenyl, C₂-C₆-alkenyl substituted by one or more hydroxyl groups, or C₁-C₆-alkyl substituted by = 0;
- X: represents O, (H,OH), (H,H) or - CH₂O-;
- Y: represents O, (H,OH), (H,H) or N-NR¹¹R¹² or N-OR¹³;
- R¹¹ and R¹²: independently represent H, C₁-C₆-alkyl, aryl selected from phenyl, tolyl, xylyl, cumenyl, mesityl and naphthyl or tosyl;
- R¹³, R¹⁴, R¹⁵, R¹⁶, R¹⁷, R¹⁸, R¹⁹, R²² and R²³: independently represent H or C₁-C₆-alkyl;
- R²⁰ and R²¹: independently represent O, or they may independently represent (R²⁰a, H) and (R²¹a, H) respectively; R²⁰a und R²¹a independently represent OH, O-(C₁-C₆)-alkyl or OCH₂OCH₂CH₂OCH₃ or R²¹ a is protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy,
in addition, R²⁰a and R²¹a may together represent an oxygen atoms in an epoxide ring;
- n: is 1 or 2;
in addition to their significances above, Y, R¹⁰ and R²³, together with the carbon atoms to which they are attached, may represent a heterocyclic ring selected from pyrrolyl and tetrahydrofuryl, and which may be substituted by one or more groups selected from C₁-C₆-alkyl, hydroxy, C₁-C₆-alkyl substituted by one or more hydroxyl groups, O-(C₁-C₆)alkyl, benzyl and -CH₂Se(C₆H₅).

The compound (I) and its pharmaceutically acceptable salt have remarkable immunosuppressive, antimicrobial and other pharmacologic activities and are known to be of value in the treatment and prevention of resistance to organ or tissue transplantation, graft-versus-host disease, various autoimmune disease and infectious disease (JP-A- 61-148181/1986 and EPA-0323042).

Such compound (I) and its pharmaceutically acceptable salt are prepared in the same manner as the one described in the above-mentioned two patent applications. Particularly, the macrolides, which are produced by fermentation of Streptomyces tsukubaensis No. 9993 (FERM BP-927) or Streptomyces hygroscopicus subsp. yakushimaensis No. 7238 (FERM BP-928), are numbered FR-900506, FR-900520, FR-900523 and FR-900525.

It is considered to prepare various kind forms of medicine such as powder, suspension, pharmaceutical solution which contains the compound (I) and pharmaceutically acceptable salt thereof (hereinafter the term "compound (I)" is representatively used for both of them). However, it is difficult to prepare a stable pharmaceutical solution of compound (I), which causes difficulties in applying the compound (I) for clinical uses where it is desired to prepare pharmaceutical solutions, e.g., injection, oral administrating liquid, local scattering solution, and dropping lotion in the eye.

It is the object of the present invention to prepare a pharmaceutical solution containing the compound (I), in particular a clear pharmaceutical solution which shows clear aqueous solution state that is especially desired for intravenous injection.

Subject-matter of the present invention is according to its first aspect a pharmaceutical solution comprising
a compound of the general formula (I) as defined above,
or a pharmaceutically acceptable salt thereof,
a pharmaceutically acceptable surface active agent and a pharmaceutically acceptable nonaqueous solvent.

According to preferred embodiments of the present invention
the compound (I) or a pharmaceutically acceptable salt thereof and the pharmaceutically acceptable surface active agent are present in a weight ratio of 1:1 to 1:100;
the pharmaceutical acceptable surface active agent is preferably a castor oil-surface active agent; and
the pharmaceutically acceptable non-aqueous solvent is preferably ethyl alcohol.

A particularly preferred compound of formula (I) is 17-allyl-1,14-dihydroxy-12-[2-(4-hydroxy-3-methoxycyclohexyl)-1-methylvinyl ]-23, 25-dimethoxy-13,19,21,27-tetramethyl-11, 28-dioxa-4-azatricyclo-[ 22.3.1.0^{4.9} ] octacos-18-ene-2,3,10,16-tetraone.

According to its second aspect the present invention relates to a process for preparing a pharmaceutical solution as defined above which is characterized by dissolving a compound of the formula (I) as defined above, or a pharmaceutically acceptable salt thereof, and a pharmaceutically acceptable surface active agent in a pharmaceutically acceptable nonaqueous solvent.

Suitable "alkyl" means straight or branched saturated aliphatic hydrocarbon residue and may include C₁-C₆-alkyl such as methyl, ethyl, propyl, isopropyl, butyl, isobutyl, pentyl, neopentyl and hexyl.

Suitable "alkenyl" means straight or branched unsaturated aliphatic hydrocarbon residue having one double bond and may include C₂-C₆-alkenyl such as vinyl, propenyl, butenyl, methylpropenyl, pentenyl and hexenyl.

Suitable "aryl" may include phenyl, tolyl, xylyl, cumenyl, mesityl and naphthyl.

Suitable examples of the protective group in the "protected hydroxyl group" may include: 1-(C₁-C₆-alkylthio)(C₁-C₆)alkyl groups such as C₁-C₆)alkylthiomethyl groups (e.g. methylthiomethyl, ethylthiomethyl, propylthiomethyl, isopropylthiomethyl, butylthiomethyl, isobutylthiomethyl and hexylthiomethyl), more desirably C₁-C₄-alkylthiomethyl groups, and most desirably methylthiomethyl; and tri-substituted silyl groups such as tri(C₁-C₆)-alkylsilyl groups (e.g. trimethylsilyl, triethylsilyl, tributylsilyl, tert-butyl-dimethylsilyl and tri-tert-butylsilyl); C₁-C₆-alkyl-diarylsilyl groups (e.g. methyldiphenylsilyl, ethyldiphenylsilyl, propyldiphenylsilyl, and tert-butyldiphenylsilyl), more desirably tri(C₁-C₄)alkylsilyl and C₁-C₄ alkyldiphenylsilyl groups and most desirably tert-butyldimethylsilyl and tert-butyldiphenylsilyl; and acyl groups such as aliphatic acyl groups, aromatic acyl groups and aliphatic acyl groups substituted by aromatic groups, which are derived from carboxylic acids, sulfonic acids or carbamic acids.

The aliphatic acyl group may includes C₁-C₆-alkanoyl groups which may optionally have one or more suitable substituents such as carboxy (e.g. formyl, acetyl, propionyl,butyryl, isobutyryl, valeryl, isovaleryl, pivaloyl, hexanoyl, carboxyacetyl, carboxypropionyl, carboxybutyryl, and carboxyhexanoyl), cyclo(C₁-C₆)alkoxy - (C₁-C₆)alkanoyl groups which may optionally have one or more appropriate substituents such as C₁-C₆-alkyl (e.g. cyclopropyloxyacetyl, cyclobutyloxypropionyl, cycloheptyloxybutyryl, menthyloxyacetyl, menthyloxypropionyl, menthyloxybutyryl, menthyloxypentanoyl, and menthyloxyhexanoyl), camphorsulfonyl, C₁-C₆-alkylcarbamoyl groups having one or more suitable substituents such as carboxy or protected carboxy, for example carboxy(C₁-C₆)alkylcarbamoyl groups( e.g. carboxymethylcarbamoyl, carboxyethylcarbamoyl, carboxypropylcarbamoyl, carboxybutylcarbamoyl, carboxypentylcarbamoyl, and carboxyhexylcarbamoyl,), and protected carboxy(C₁-C₆)alkylcarbamoyl groups such as tri(C₁-C₆)alkylsilyl(C₁-C₆)alkoxycarbonyl(C₁-C₆)alkylcarbamoyl groups (e.g. trimethylsilylmethoxycarbonylethylcarbamoyl, trimethylsilylethoxycarbonylpropylcarbamoyl, triethylsilylethoxycarbonylpropylcarbamoyl, tert-butyldimethylsilylethoxycarbonylpropylcarbamoyl, and trimethylsilylpropoxycarbonylbutylcarbamoyl).

The aromatic acyl group may include aroyl groups which may optionally have one or more suitable substituents such as nitro (e.g. benzoyl, toluoyl, xyloyl, naphthoyl, nitrobenzoyl, dinitrobenzoyl, and nitronaphthoyl), and arenesulfonyl groups which may optionally have one or more suitable substituent(s) such as halogen (e.g.benzenesulfonyl, toluenesulfonyl, xylenesulfonyl, naphthalenesulfonyl, fluorobenzenesulfonyl, chlorobenzenesulfonyl, bromobenzenesulfonyl, and iodobenzenesulfonyl).

The aromatic group-substituted aliphatic acyl groups may include ar(C₁-C₆)alkanoyl groups which may optionally have one or more suitable substituent(s) such as C₁-C₆-alkoxy and trihalo(C₁-C₆)alkyl (e.g. phenylacetyl, phenylpropionyl, phenylbutyryl, 2-trifluoromethyl-2-methoxy-2-phenylacetyl, 2-ethyl-2-trifluoromethyl-2-phenylacetyl, and 2-trifluoromethyl-2-propoxy-2-phenylacetyl).

Among the above-mentioned acyl groups, the more desirable acyl groups are C₁-C₄ alkanoyl groups which may optionally be substituted by carboxy, cyclo(C₅-C₆)alkyloxy(C₁-C₄)alkanoyl groups having two (C₁-C₄)alkyl groups in the cycloalkyl moiety, camphorsulfonyl, carboxy(C₁ -C₄ )alkylcarbamoyl groups, tri (C₁-C₄) alkylsilyl(C₁-C₄)alkoxycarbonyl(C₁-C₄)alkylcarbamoyl groups, benzoyl which may have one or two nitro groups, halogen-substituted benzenesulfonyl groups, phenyl(C₁-C₄)alkanoyl groups having C₁ - C₄ alkoxy and trihalo(C₁ - C₄)alkyl groups. Of these groups, the most desirable are acetyl, carboxypropionyl, menthyloxyacetyl, camphorsulfonyl, benzoyl, nitrobenzoyl, dinitrobenzoyl, iodobenzenesulfonyl and 2-trifluoromethyl-2-methoxy-2-phenylacetyl.

Suitable "5- or 6-membered N-, S- or O- containing heterocyclic ring "may include pyrrolyl, and tetrahydrofuryl.

The pharmaceutically acceptable salt of compound (I) is a nontoxic salt, which may be the corresponding salt with an inorganic or organic base such as alkali metal salts (e.g. sodium salt, and potassium salt), ammonium salt and amine salts (e.g. triethylamine salt, and N-benzyl-N-methylamine salt).

With regard to the compound (I) of the present invention, it is to be noted that there may be one or more than one conformers or stereoisomers such as optically or geometrically isomeric pairs due to the presence of one or are than one asymmetric carbon atom or double bond, and these are included within a scope of the compound (I) of the present invention.

It will hereinafter be described in detail how the present invention has completed, especially relating to the important point of the present invention i.e., the reason why a mixture of surface active agent and nonaqueous solvent is selected in this invention.

A liquidous pharmaceutical containing the compound (I) should be offered as a stable liquid as it is for the purpose of administrating to human body and transferring the effective amount of the ingredient compound to human body. Further on considering a special use such as intravenous injection that is the main object of the present invention, should be offered a whole clear liquidous pharmaceutical which can maintain the clearity under long term storage.

From the above point of view, the inventors of the present invention studied, at first, the solubility of the compound (I) in water. As a test compound, the inventors selected the following compound as a free form which has an excellent immunosupressive activity and is called FK 506 hereafter.
- R¹, R², R⁸, R²³ = hydrogen: R⁷, R⁹ = hydroxy
- R¹⁴, R¹⁵, R¹⁶, R¹⁷, R¹⁸, R¹⁹, R²² = methyl: R¹⁰ = allyl
- R²⁰ = R²⁰a,H (R²⁰a = methoxy): X, Y = oxygen
- R²¹ = R²¹a,H (R²¹a = hydroxy): n = 2

- R³, R⁴ =: form a second bond between the vicinal carbon atoms to which they are attached
- R⁵, R⁶ =: form a second bond between the vicinal carbon atoms to which they are attached

The solubility of FK 506 in water is at most 3 µg/ml under ambient temperature. Accordingly, it is decided to add a surface active agent in order to increase the solubility of FK 506 in water to the level where the clinically effective amount of FK 506 is dissolved. Table 1 shows the solubility of FK 506 under the different condition e.g., the kind and the concentration of a surface active agent and temperature. As a surface active agent, a castor oil-surface active agent i.e., HCO-10, HCO-40, HCO-60 (trademark, prepared by Nikko Chemicals, respectively) are selected for examination.

It is conjectured from the result shown in Table 1 that the concentration of the surface active agent should be controlled at 1.43w/v% [about 150mg of the surface active agent against 1mg of FK506] to dissolve 0.1mg of FK 506 in 1ml of water, calculating based on the result that 0.035mg of FK 506 is dissolved in 1ml of 0.5w/v% HCO-60 aqueous solution at 20 °C. Accordingly, if it is desired to prepare 5mg/ml aqueous solution FK 506, the concentration of the surface active agent is estimated to come up to 87w/v% from the calculation based on the result of HCO-60, 20w/v%, 20°C. Such tolerably high concentration of surface active agent in aqueous solution should not be realized in practice of clinical field.

**Table 1**

| Concentration of surface active agent (w/v %) | Kind of surface active agent and solubility of FK506 (mg/ml) | | | | |
|---|---|---|---|---|---|
| | HCO-40 | HCO-60 | | Mixture of HCO-60 and HCO-10 (4:1) | |
| | 20 °C | 20°C | 30°C | 20°C | 30°C |
| 0.1 | ― | 0.005 | ― | ― | ― |
| 0.3 | ― | 0.019 | ― | ― | ― |
| 0.5 | ― | 0.035 | ― | ― | ― |
| 5 | 0.40 | 0.28 | 0.29 | 0.26 | 0.27 |
| 10 | 0.78 | 0.61 | 0.57 | 0.56 | 0.56 |
| 20 | 1.52 | 1.15 | 1.14 | 1.13 | 1.14 |
| HCO-60 : Polyoxyethylenehydrogenated Castor Oil 60 HCO-40 : Polyoxyethylenehydrogenated Castor Oil 40 HCO-10 : Polyoxyethylenehydrogenated Castor Oil 10 | | | | | |

Table 2 shows a remaining percentage of FK 506 in aqueous solution wherein FK 506 is dissolved in water in the presence of large amount of surface active agent. From this table, it is found that the stability for long period storage is not expectable in the above prescription .

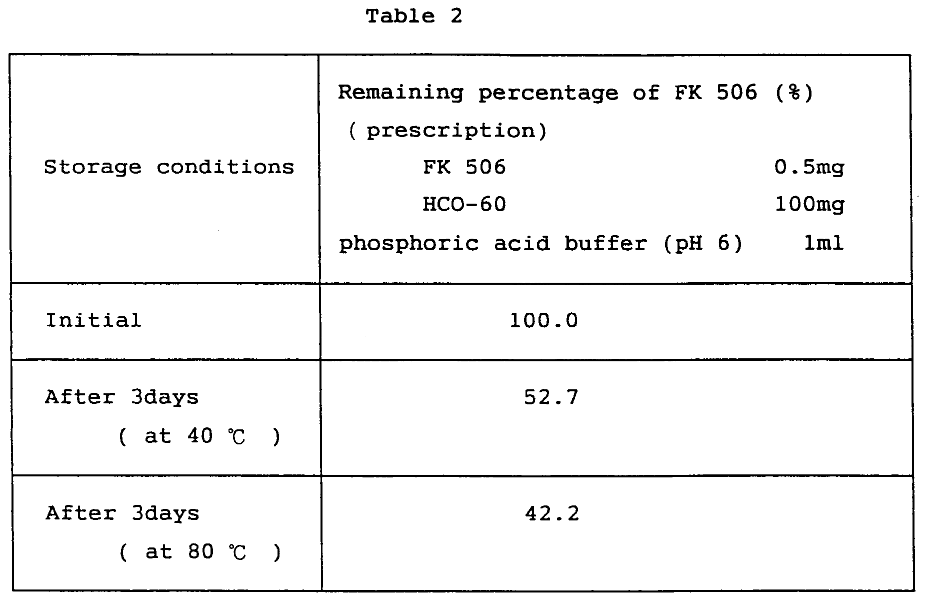

From the results of the above experiment, it is recognized that the use of surface active agent is not profitable means for the purpose of dissolving the compound (I), for example FK 506, in water.

Table 3 shows solubility of FK 506 in several kinds of nonaqueous solvent such as PEG (polyethylene glycol) 400, ethanol and propylene glycol. From the consideration of the illustrated data, it is found that FK 506 is dissolved in the experimented solvent at the concentration of more than 40mg/ml.

**Table 3**

| Solvent | Solubility (at ambient temperature) |
|---|---|
| Ethanol | >300 |
| PEG 400 | >40 |
| Propylene glycol | >40 |
| (mg/ml) | |

Nonaqueous solvent is, at the time of administrating into the human vascular tract, usually diluted with aqueous solvent such as physiological saline, since nonaqueous solvent has hemolysis action. Therefore, the inventors of the present invention diluted the experimental solution by using nonaqueous solution (1ml) described in the following prescriptions 1 & 2 with physiological saline (100ml), and it was found that the nonaqueous solution became turbid at once and fine crystal of FK 506 was precipitated in a mixed medium.

| (Prescription 1) | |
|---|---|
| FK 506 | 10mg |
| Ethanol | to 1ml |

| (Prescription 2) | |
|---|---|
| FK 506 | 10mg |
| Propylene glycol | to 1ml |

Standing on the above result, the inventors of the present invention have then studied on combination use of nonaqueous solvent and surface active agent.

The experimental solution (1ml) of the following prescription 3 consisting of FK 506, surface active agent and nonaqueous solvent was diluted with physiological saline (100ml) to find that the clear appearance of prescription was kept unchanged.

| (Prescription 3) | |
|---|---|
| FK 506 | 10mg |
| HCO-60 | 100mg |
| Ethanol | to 1ml |

Then several prescriptions of clear solution were further prepared altering the concentration of FK 506, the kind and the concentration of surface active agent, and were tested how change the clearity of the solution and whether separate out the crystal or not under the several condition of different degree of dilution. The results thereof are shown in Table 4.

**Table 4**

| Concentration of FK 506 (mg/ml) | Dilution times with physiological saline | Period till solution becomes turbid to crystallize FK506 (in days) | | | | | | |
|---|---|---|---|---|---|---|---|---|
| | | Kind of surface active agent/Ratio of ethanol to surface active agent | | | | | | |
| | | HCO-60 | | | Cremophor®EL | | HCO-40 | |
| | | 35/65 | 60/40 | 80/20 | 35/65 | 80/20 | 40/60 | 80/20 |
| 5 | 2 | ≧7 | ≧7 | ≧7 | ≧7 | ≧7 | ≧7 | ≧7 |
| | 100 | ≧7 | ≧7 | ≧7 | ≧7 | ≧7 | ≧7 | ≧7 |
| 10 | 2 | ≧7 | ≧7 | ≧7 | ≧7 | ≧7 | ≧7 | ≧7 |
| | 100 | ≧7 | ≧7 | ≧7 | ≧7 | ≧7 | ≧7 | ≧7 |
| 25 | 2 | ≧7 | ≧7 | ≧7 | ≧7 | ≧7 | ≧7 | ≧7 |
| | 100 | ≧7 | ≧1 | ≧7 | ≧7 | ≧1 | ≧7 | ≧1 |
| 50 | 2 | ≧7 | ≧7 | ≧7 | ≧7 | ≧1 | ≧7 | ≧1 |
| Cremophor®EL : trademark, prepared by BASF (Polyoxyethylene Castor Oil 35) | | | | | | | | |

It is concluded from the result illustrated in Table 4 that a clear pharmaceutical solution which does not cause precipitation of the compound (I) on diluting with physiological saline can be prepared by controlling the ratio of the compound(I), surface active agent and nonaqueous solvent according to the kind of surface active agent under the condition that the concentration of the compound (I) is less than 50mg/ml.

Lastly it is tested the remaining percentage of FK 506 after storage in nonaqueous solvent containing both FK 506 and surface active agent. In the test solution, the concentration of FK 506 is adjusted to 5mg/ml, and, for comparative a nonaqueous solution which contain only FK 506 is prepared. The experimental result is shown in Table 5.

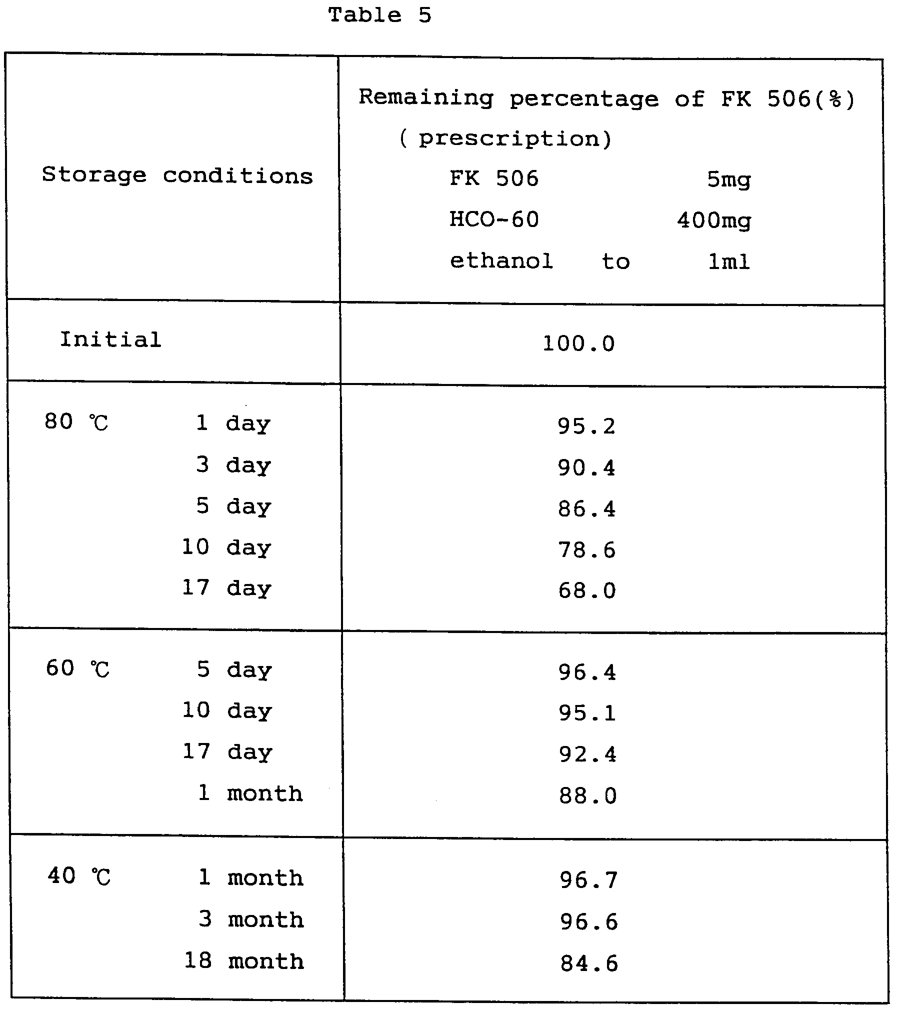

It is concluded from the result shown in table 5 that HCO-60 is the most preferable surface active agent in the view point of storage stability.

As considered from the above experiments, the compound (I) such as FK 506 has quite poor solubility in water and this is not so improved even in the presence of surface active agent, and the storage stability especially at ambient temperature is quite inferior excepting that only frozen one can stand for some period of time.

In the mean-time, it is found that the compound (I) is well dissolved in nonaqueous solvent. However it causes precipitation of the compound (I) on diluting with physiological saline to diminishing hemolysis action of nonaqueous solvent. Occurence of precipitation makes it impossible to use in the clinical field.

Under the condition of cooperative use of nonaqueous solvent and surface active agent, it is noted that the compound (I) is well dissolved and there is no problem after long period storage, and further it does not happen to give any precipitate at the time of diluting with physiological saline.

The kind of nonaqueous solvent is not limited in the present invention, and any nonaqueous solvent may be used as much as it can dissolve the effective amount of the compound (I) and it may be acceptable in clinical use. The nonaqueous solvent may be used either alone or as a mixture thereof. Suitable examples thereof include ethanol, propylene glycol, glycerin, polyethylene glycol (e.g., PEG 400, PEG 300, and PEG 200), or the mixture thereof from a view point of solubility and viscosity, and most preferable one is ethanol. The representative examples of the surface active agent include, from a view point of storage stability for long term, a castor oil-surface active agents, and more preferable one is HCO (polyoxyethylene hardened oil)-surface active agents, and most preferable one is HCO-6O, and HCO-5O. In addition to the above exemplified surface active agents, polyoxyethylene sorbitane fatty acid ester-derivative (e.g., Polysorbate 80), glycerine fatty acid ester derivative (e.g., glycerine monocaprylate), polyethylene glycol fatty acid ester derivative (e.g., polyoxyethylene 40 monostearate), and the like may also be used.

The concentration of the compound (I) is determined from the judgement including the kind and the concentration of nonaqueous solvent and surface active agent, the composition ratio thereof, the stability after being diluted with physiological saline, etc. and storage stability. The suitable range of thus determined concentration is usually in the range of 0.1∼ 50mg/ml and more preferably 1 ∼ 20mg/ml.

As for the amount of surface active agent, it is noted to be less than the calculated estimation. From the experimental calculation based on the result illustrated in Table 1, about 150mg of the surface active agent may be necessary to obtain a saturated aqueous solution containing 1 mg of the compound (I) as stated above. However in the present invention the compound (I) is dissolved in a mixed solution of nonaqueous solvent - surface active agent - water to form stable supersaturation state, and therefore the necessary amount of surface active agent becomes less than the calculated one. These specific action, i.e., the low precipitating speed of thecrystalline from the supersaturated solution, is based on the characteristic property of the compound (I). The range of the ratio of surface active agent to the compound (I) is preferably 1∼ 100mg/1mg, and more preferably 30∼ 60mg/1mg to prevent the occurence of precipitation at the time of diluting for clinical use.

The pharmaceutical solution of the present invention may further contain, if necessary, other agent such as stabilizing agent, and anodyne.

The pharmaceutical solution of the present invention is stable during long term storage and does not occur the precipitation of crystalline at the time of dilution. Therefore, this is applicable to various kind of medicine form such as intravenous injection, dropping lotion in the eye, dropping lotion in the nose, intraenteric injection, percutaneous liniment, local scattering agent, and oral administration agent (e.g., syrup).

### Example

Following prescriptions are shown only for the purpose of the explanation of this invention.

| Prescription 1 | |
|---|---|
| FK 506 | 10mg |
| HCO-60 | 400mg |
| Ethanol | to 1ml |

The solution comprising the ingredients stated above is prepared by dissolving the FK 506 and HCO-60 in ethanol by a conventional manner.

The following solutions are also prepared a similar manner of the Prescription 1.

| Prescription 2 | |
|---|---|
| FK 506 | 5mg |
| HCO-40 | 200mg |
| PEG 400 | to 1ml |

| Prescription 3 | |
|---|---|
| FK 506 | 2mg |
| Polysorbate 80 | 50mg |
| Propylene glycol | to 1ml |

| Prescription 4 | |
|---|---|
| FK 506 | 2mg |
| Polysorbate 80 | 10mg |
| Glycerine | 0.5ml |
| Ethanol | to 1ml |

| Prescription 5 | |
|---|---|
| FK 506 | 2mg |
| HCO-60 | 20mg |
| Propylene glycol | to 1ml |

| Prescription 6 | |
|---|---|
| FK 506 | 1mg |
| Polyoxyethylene (40) mono stearate | 20mg |
| Propylene glycol | to 1ml |

| Prescription 7 | |
|---|---|
| FK 506 | 10mg |
| HCO-60 | 400mg |
| Ethanol | to 1ml |

| Prescription 8 | |
|---|---|
| FK 506 | 5mg |
| HCO-60 | 400mg |
| Ethanol | to 1ml |

| Prescription 9 | |
|---|---|
| FK 506 | 25mg |
| HCO-60 | 400mg |
| Ethanol | to 1ml |

| Prescription 10 | |
|---|---|
| FK 506 | 2mg |
| HCO-60 | 10mg |
| Glycerin | 0.5ml |
| Ethanol | to 1ml |

Thus obtained nonaqueous pharmaceutical solution containing the compound (I) is stable during long term storage and does not occur any precipitation at the time of diluting with physiological saline, glucose solution for injection, water, fruit juice, milk, or the like for clinical use. Accordingly, the pharmaceutical solution of the present invention is applicable to various kind of medicine form such as intravenous injection, oral administration agent and the like, which could contribute the compound (I) in clinical field wherein its immunosuppressive activity is intensely desired. Particularly, the most preferable medicine form of the present nonaqueous pharmaceutical solution is the one for intravenous injection by diluting with the physiological saline.

## Claims (Claims for the following Contracting State(s): AT, BE, CH, DE, DK, FR, GB, IT, LI, LU, NL, SE)

1. A pharmaceutical solution comprising
a compound of the general formula 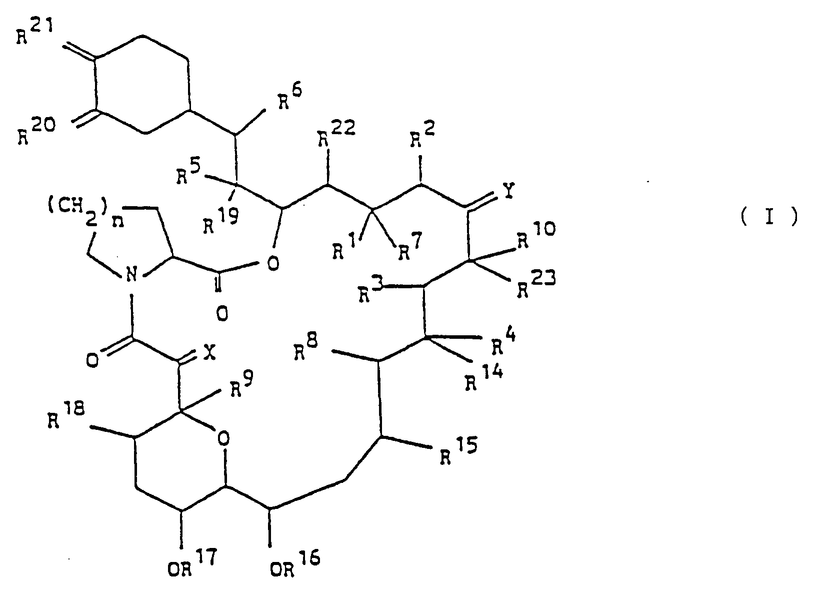 wherein each vicinal pair of substituents [R¹ and R² ], [R³ and R⁴ ], [R⁵ and R⁶] independently
a) represent two vicinal hydrogen atoms, or
b) form a second bond between the vicinal carbon atoms to which they are attached;
in addition to its significance above, R² may represent a C₁-C₆-alkyl group;
R⁷ represents H, OH, protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy, or O-(C₁-C₆)alkyl,
R⁸ and R⁹ independently represent H or OH;
R¹⁰ represents H, C₁-C₆-alkyl, C₁-C₆-alkyl substituted by one or more hydroxyl groups, C₂-C₆-alkenyl, C₂-C₆-alkenyl substituted by one or more hydroxyl groups, or C₁-C₆-alkyl substituted by = 0;
X represents O, (H,OH), (H,H) or - CH₂O-;
Y represents O, (H,OH), (H,H) or N-NR¹¹R¹² or N-OR¹³;
R¹¹ and R¹² independently represent H, C₁-C₆-alkyl, aryl selected from phenyl, tolyl, xylyl, cumenyl, mesityl and naphthyl or tosyl;
R¹³, R¹⁴, R¹⁵, R¹⁶, R¹⁷, R¹⁸, R¹⁹, R²² and R²³ independently represent H or C₁-C₆-alkyl;
R²⁰ and R²¹ independently represent O, or they may independently represent (R²⁰a, H) and (R²¹a, H) respectively; R²⁰a und R²¹a independently represent OH, O-(C₁-C₆)-alkyl or OCH₂OCH₂CH₂OCH₃ or R²¹ a is protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy,
in addition, R²⁰a and R²¹a may together represent an oxygen atoms in an epoxide ring;
n is 1 or 2;
in addition to their significances above, Y, R¹⁰ and R²³, together with the carbon atoms to which they are attached, may represent a heterocyclic ring selected from pyrrolyl and tetrahydrofuryl, and which may be substituted by one or more groups selected from C₁-C₆-alkyl, hydroxy, C₁-C₆-alkyl substituted by one or more hydroxyl groups, O-(C₁-C₆)alkyl, benzyl and - CH₂Se(C₆H₅);
or a pharmaceutically acceptable salt thereof,
a pharmaceutically acceptable surface active agent and a pharmaceutically acceptable nonaqueous solvent.

2. The pharmaceutical solution of claim 1, wherein the compound (I) or a pharmaceutically acceptable salt thereof and the pharmaceutically acceptable surface active agent are present in a weight ratio of 1:1 to 1:100.

3. The pharmaceutical solution of claim 2, wherein the pharmaceutically acceptable surface active agent is a castor oil-surface active agent.

4. The pharmaceutical solution of claim 3, wherein the pharmaceutically acceptable nonaqueous solvent is ethyl alcohol.

5. The pharmaceutical solution of claim 4, wherein the compound (I) is 17-allyl-1,14-dihydroxy-12-[2-(4-hydroxy-3-methoxycyclohexyl)-1-methylvinyl ]-23, 25-dimethoxy-13,19,21,27-tetramethyl-11, 28-dioxa-4-azatricyclo-[ 22.3.1.0^{4.9} ] octacos-18-ene-2,3,10,16-tetraone.

6. A process for preparing a pharmaceutical solution, which is characterized by dissolving a compound of the formula: 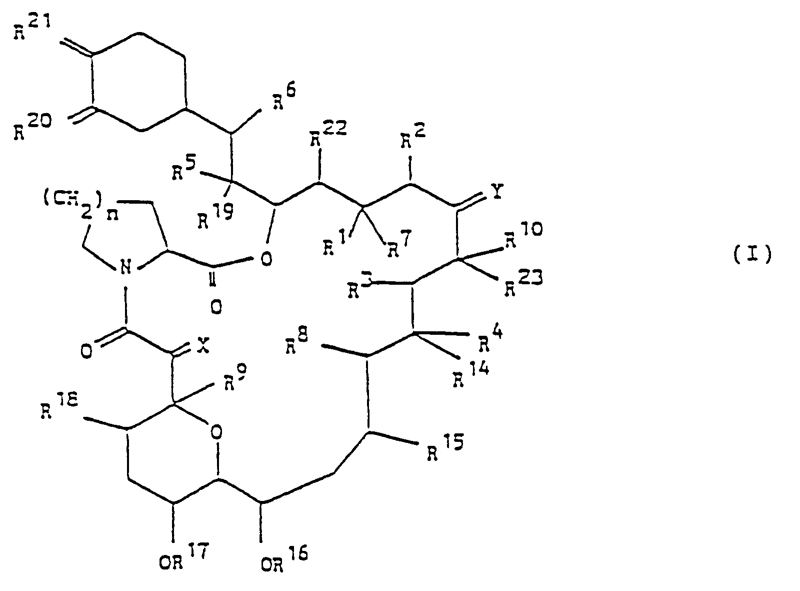 wherein each vicinal pair of substituents [R¹ and R² ], [R³ and R⁴ ], [R⁵ and R⁶] independently
a) represent two vicinal hydrogen atoms, or
b) form a second bond between the vicinal carbon atoms to which they are attached;
in addition to its significance above, R² may represent a C₁-C₆-alkyl group;
R⁷ represents H, OH, protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy, or O-(C₁-C₆)alkyl,
R⁸ and R⁹ independently represent H or OH;
R¹⁰ represents H, C₁-C₆-alkyl, C₁-C₆-alkyl substituted by one or more hydroxyl groups, C₂-C₆-alkenyl, C₂-C₆-alkenyl substituted by one or more hydroxyl groups, or C₁-C₆-alkyl substituted by = 0;
X represents O, (H,OH), (H,H) or - CH₂O-;
Y represents O, (H,OH), (H,H) or N-NR¹¹R¹² or N-OR¹³;
R¹¹ and R¹² independently represent H, C₁-C₆-alkyl, aryl selected from phenyl, tolyl, xylyl, cumenyl, mesityl and naphthyl or tosyl;
R¹³, R¹⁴, R¹⁵, R¹⁶, R¹⁷, R¹⁸, R¹⁹, R²² and R²³ independently represent H or C₁-C₆-alkyl;
R²⁰ and R²¹ independently represent O, or they may independently represent (R²⁰a, H) and (R²¹a, H) respectively; R²⁰a und R²¹a independently represent OH, O-(C₁-C₆)-alkyl or OCH₂OCH₂CH₂OCH₃ or R²¹a is protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy,
in addition, R²⁰a and R²¹a may together represent an oxygen atoms in an epoxide ring;
n is 1 or 2;
in addition to their significances above, Y, R¹⁰ and R²³, together with the carbon atoms to which they are attached, may represent a heterocyclic ring selected from pyrrolyl and tetrahydrofuryl, and which may be substituted by one or more groups selected from C₁-C₆-alkyl, hydroxy, C₁-C₆-alkyl substituted by one or more hydroxyl groups, O-(C₁-C₆)alkyl, benzyl and -CH₂Se(C₆H₅);
or a pharmaceutically acceptable salt thereof, and
a pharmaceutically acceptable surface active agent in a pharmaceutically acceptable nonaqueous solvent.

## Claims (Claims for the following Contracting State(s): ES, GR)

1. A process for preparing a pharmaceutical solution, which is characterized by dissolving a compound of the formula: 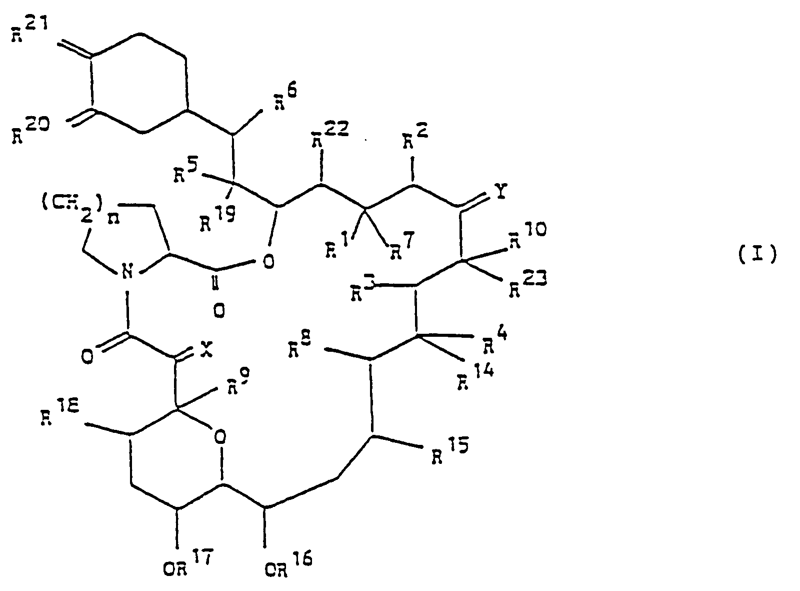 wherein each vicinal pair of substituents [R¹ and R² ], [R³ and R⁴ ], [R⁵ and R⁶] independently
a) represent two vicinal hydrogen atoms, or
b) form a second bond between the vicinal carbon atoms to which they are attached;
in addition to its significance above, R² may represent a C₁-C₆-alkyl group;
R⁷ represents H, OH, protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy, or O-(C₁-C₆)alkyl,
R⁸ and R⁹ independently represent H or OH;
R¹⁰ represents H, C₁-C₆-alkyl, C₁-C₆-alkyl substituted by one or more hydroxyl groups, C₂-C₆-alkenyl, C₂-C₆-alkenyl substituted by one or more hydroxyl groups, or C₁-C₆-alkyl substituted by = 0;
X represents O, (H,OH), (H,H) or - CH₂O-;
Y represents O, (H,OH), (H,H) or N-NR¹¹R¹² or N-OR¹³;
R¹¹ and R¹² independently represent H, C₁-C₆-alkyl, aryl selected from phenyl, tolyl, xylyl, cumenyl, mesityl and naphthyl or tosyl;
R¹³, R¹⁴, R¹⁵, R¹⁶, R¹⁷, R¹⁸, R¹⁹, R²² and R²³ independently represent H or C₁-C₆-alkyl;
R²⁰ and R²¹ independently represent O, or they may independently represent (R²⁰a, H) and (R²¹a, H) respectively; R²⁰a und R²¹a independently represent OH, O-(C₁-C₆)-alkyl or OCH₂OCH₂CH₂OCH₃ or R²¹a is protected hydroxy selected from 1-(C₁-C₆-alkylthio)(C₁-C₆)alkyloxy, tri(C₁-C₆)alkylsilyloxy, C₁-C₆-alkyl-diphenyl-silyloxy and acyloxy,
in addition, R²⁰a and R²¹a may together represent an oxygen atoms in an epoxide ring;
n is 1 or 2;
in addition to their significances above, Y, R¹⁰ and R²³, together with the carbon atoms to which they are attached, may represent a heterocyclic ring selected from pyrrolyl and tetrahydrofuryl, and which may be substituted by one or more groups selected from C₁-C₆-alkyl, hydroxy, C₁-C₆-alkyl substituted by one or more hydroxyl groups, O-(C₁-C₆)alkyl, benzyl and -CH₂Se(C₆H₅);
or a pharmaceutically acceptable salt thereof, and
a pharmaceutically acceptable surface active agent in a pharmaceutically acceptable nonaqueous solvent.

2. The process of claim 1, wherein the compound (I) or a pharmaceutically acceptable salt thereof and the pharmaceutically acceptable surface active agent are present in a weight ratio of 1:1 to 1:100.

3. The process of claim 2, wherein the pharmaceutically acceptable surface active agent is a castor oil-surface active agent.

4. The process of claim 3, wherein the pharmaceutically acceptable nonaqueous solvent is ethyl alcohol.

5. The process of claim 4, wherein the compound (I) is 17-allyl-1,14-dihydroxy-12-[2-(4-hydroxy-3-methoxycyclohexyl)-1-methylvinyl ]-23, 25-dimethoxy-13,19,21,27-tetramethyl-11, 28-dioxa-4-azatricyclo-[ 22.3.1.0^{4.9} ]octacos-18-ene-2,3,10,16-tetraone.

## Patentansprüche (Patentansprüche für folgende(n) Vertragsstaat(en): AT, BE, CH, DE, DK, FR, GB, IT, LI, LU, NL, SE)

1. Pharmazeutische Lösung, die umfaßt: Eine Verbindung der allgemeinen Formel: 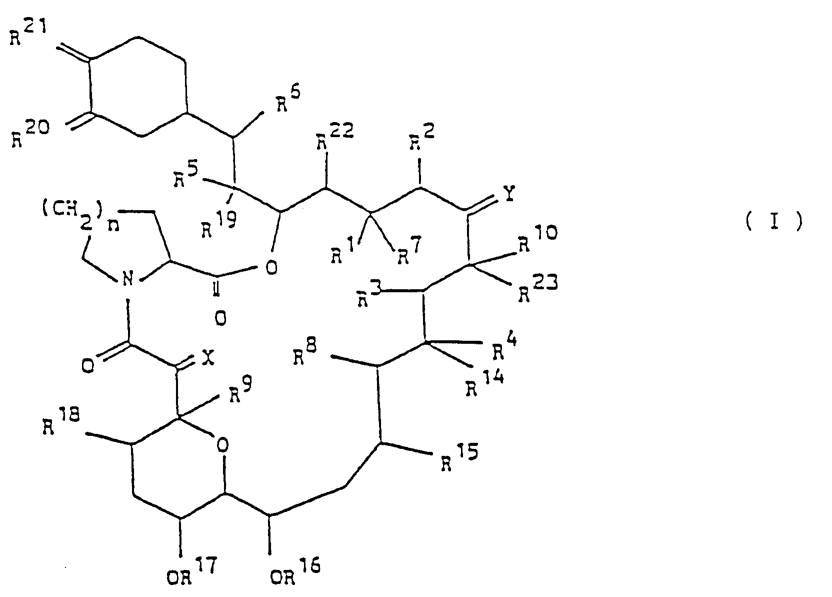 worin jedes vizinale Paar von Substituenten [R¹ und R²], [R³ und R⁴], [R⁵ und R⁶] unabhängig voneinander
a) zwei vizinale Wasserstoffatome darstellt, oder
b) eine zweite Bindung zwischen den vizinalen Kohlenstoffatomen bilden, an die sie gebunden sind;
zusätzlich zu seiner obigen Bedeutung R² eine (C₁-C₆)-Alkylgruppe darstellen kann;
R⁷ H, OH, geschütztes Hydroxy, ausgewählt aus 1-(C₁-C₆)-Alkylthio)(C₁-C₆)alkyloxy, Tri(C₁-C₆)alkylsilyloxy, (C₁-C₆)Alkyl-diphenyl-silyloxy und Acyloxy, oder O-(C₁-C₆)Alkyl darstellt,
R⁸ und R⁹ unabhängig voneinander H oder OH darstellen;
R¹⁰ H, (C₁-C₆)Alkyl, (C₁-C₆)Alkyl, substituiert durch eine oder mehrere Hydroxylgruppen, (C₂-C₆)Alkenyl, (C₂-C₆)-Alkenyl, substituiert durch eine oder mehrere Hydroxylgruppen, oder (C₁-C₆)-Alkyl, substituiert durch =O, darstellt;
X O, (H,OH), (H,H) oder - CH₂O- darstellt;
Y O, (H,OH), (H,H) oder N-NR¹¹R¹² oder N-OR¹³ darstellt;
R¹¹ und R¹² unabhängig voneinander H, (C₁-C₆)Alkyl, Aryl, ausgewählt aus Phenyl, Tolyl, Xylyl, Cumenyl, Mesityl und Naphthyl, oder Tosyl darstellen;
R¹³, R¹⁴ R¹⁵, R¹⁶, R¹⁷, R¹⁸, R¹⁹, R²² und R²³ unabhängig voneinander H oder (C₁-C₆)Alkyl darstellen;
R²⁰ und R²¹ unabhängig voneinander O darstellen, oder abhängig voneinander (R²⁰a, H) beziehungsweise (R²¹a, H) darstellen; R²⁰a und R²¹a unabhängig voneinander OH, O-(C₁-C₆)-Alkyl oder OCH₂OCH₂CH₂OCH₃ darstellen, oder R²¹a geschütztes Hydroxy ist, ausgewählt aus 1-(C₁-C₆)-Alkylthio(C₁-C₆)alkyloxy, Tri(C₁-C₆)alkylsilyloxy, (C₁-C₆)Alkyl-diphenyl-silyloxy und Acyloxy ist,
zusätzlich R²⁰a und R²¹a zusammen ein Sauerstoffatom in einem Epoxidring darstellen können;
n 1 oder 2 ist;
zusätzlich zu ihren obigen Bedeutungen Y, R¹⁰ und R²³ zusammen mit den Kohlenstoffatomen, an die sie gebunden sind, einen heterocyclischen Ring darstellen können, der aus Pyrrolyl und Tetrahydrofuryl ausgewählt ist, und der durch eine oder mehrere Gruppen, ausgewählt aus (C₁-C₆)Alkyl, Hydroxy, (C₁-C₆)Alkyl, substituiert durch eine oder mehrere Hydroxylgruppen, O-(C₁-C₆)Alkyl, Benzyl und -CH₂Se(C₆H₅), substituiert sein können;
oder ein pharmazeutisch verträgliches Salz davon, ein pharmazeutisch verträgliches oberflächenaktives Mittel und ein pharmazeutisch verträgliches nichtwäßriges Lösungsmittel.

2. Pharmazeutische Lösung nach Anspruch 1, worin die Verbindung (I) oder ein pharmazeutisch verträgliches Salz davon und das pharmazeutisch verträgliche oberflächenaktive Mittel in einem Gewichtsverhältnis von 1:1 bis 1:100 vorliegen.

3. Pharmazeutische Lösung nach Anspruch 2, worin das pharmazeutisch verträgliche oberflächenaktive Mittel ein Castoröloberflächenaktives Mittel ist.

4. Pharmazeutische Lösung nach Anspruch 3, worin das pharmazeutisch verträgliche nichtwäßrige Lösungsmittel Ethylalkohol ist.

5. Pharmazeutische Lösung nach Anspruch 4, worin die Verbindung (I) 17-Allyl-1,14-dihydroxy-12-[2-(4-hydroxy-3-methoxycyclo-hexyl)-1-methylvinyl]-23,25-dimethoxy-13,19,21,27-tetramethyl- 11,28-dioxa-4-azatricyclo-[22.3.1.0^{4.9}]octacos-18-en-2,3,10,16-tetraon ist.

6. Verfahren zur Herstellung einer pharmazeutischen Lösung, das gekennzeichnet ist, durch Lösen einer Verbindung der Formel: 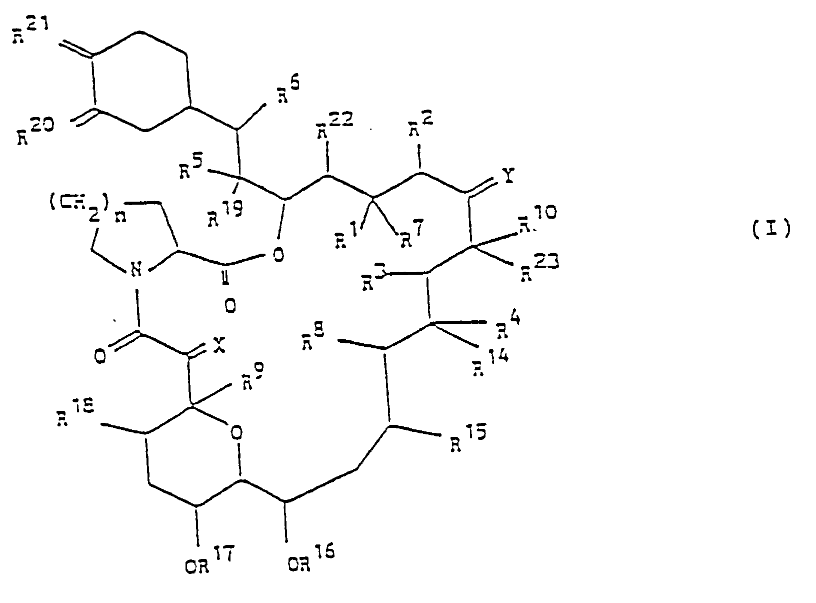 worin jedes vizinale Paar von Substituenten [R¹ und R²], [R³ und R⁴], [R⁵ und R⁶] unabhängig voneinander
a) zwei vizinale Wasserstoffatome darstellt, oder
b) eine zweite Bindung zwischen den vizinalen Kohlenstoffatomen bilden, an die sie gebunden sind;
zusätzlich zu seiner obigen Bedeutung R² eine (C₁-C₆)-Alkylgruppe darstellen kann;
R⁷ H, OH, geschütztes Hydroxy, ausgewählt aus 1-(C₁-C₆)-Alkylthio)(C₁-C₆)alkyloxy, Tri(C₁-C₆)alkylsilyloxy, (C₁-C₆)Alkyl-diphenyl-silyloxy und Acyloxy, oder O-(C₁-C₆)Alkyl darstellt,
R⁸ und R⁹ unabhängig voneinander H oder OH darstellen;
R¹⁰ H, (C₁-C₆)Alkyl, (C₁-C₆)Alkyl, substituiert durch eine oder mehrere Hydroxylgruppen, (C₂-C₆)Alkenyl, (C₂-C₆)-Alkenyl, substituiert durch eine oder mehrere Hydroxylgruppen, oder (C₁-C₆)-Alkyl, substituiert durch =O, darstellt;
X O, (H,OH), (H,H) oder - CH₂O- darstellt;
Y O, (H,OH), (H,H) oder N-NR¹¹R¹² oder N-OR¹³ darstellt;
R¹¹ und R¹² unabhängig voneinander H, (C₁-C₆)Alkyl, Aryl, ausgewählt aus Phenyl, Tolyl, Xylyl, Cumenyl, Mesityl und Naphthyl, oder Tosyl darstellen;
R¹³, R¹⁴, R¹⁵, R¹⁶, R¹⁷, R¹⁸, R¹⁹, R²² und R²³ unabhängig voneinander H oder (C₁-C₆)Alkyl darstellen;
R²⁰ und R²¹ unabhängig voneinander O darstellen, oder abhängig voneinander (R²⁰a, H) beziehungsweise (R²¹a, H) darstellen; R²⁰a und R²¹a unabhängig voneinander OH, O-(C₁-C₆)-Alkyl oder OCH₂OCH₂CH₂OCH₃ darstellen, oder R²¹a geschütztes Hydroxy ist, ausgewählt aus 1-(C₁-C₆)-Alkylthio(C₁-C₆)alkyloxy, Tri(C₁-C₆)alkylsilyloxy, (C₁-C₆)Alkyl-diphenyl-silyloxy und Acyloxy ist,
zusätzlich R²⁰a und R²¹a zusammen ein Sauerstoffatom in einem Epoxidring darstellen können;
n 1 oder 2 ist;
zusätzlich zu ihren obigen Bedeutungen Y, R¹⁰ und R²³ zusammen mit den Kohlenstoffatomen, an die sie gebunden sind, einen heterocyclischen Ring darstellen können, der aus Pyrrolyl und Tetrahydrofuryl ausgewählt ist, und der durch eine oder mehrere Gruppen, ausgewählt aus (C₁-C₆)Alkyl, Hydroxy, (C₁-C₆)Alkyl, substituiert durch eine oder mehrere Hydroxylgruppen, O-(C₁-C₆)Alkyl, Benzyl und -CH2Se(C₆H₅), substituiert sein können;
oder eines pharmazeutisch verträglichen Slazes davon, und eines pharmazeutisch verträglichen oberflächenaktiven Mittels in einem pharmazeutisch verträglichen nichtwäßrigen Lösungsmittel.

## Patentansprüche (Patentansprüche für folgende(n) Vertragsstaat(en): ES, GR)

1. Verfahren zur Herstellung einer pharmazeutischen Lösung, das gekennzeichnet ist, durch Lösen einer Verbindung der Formel: 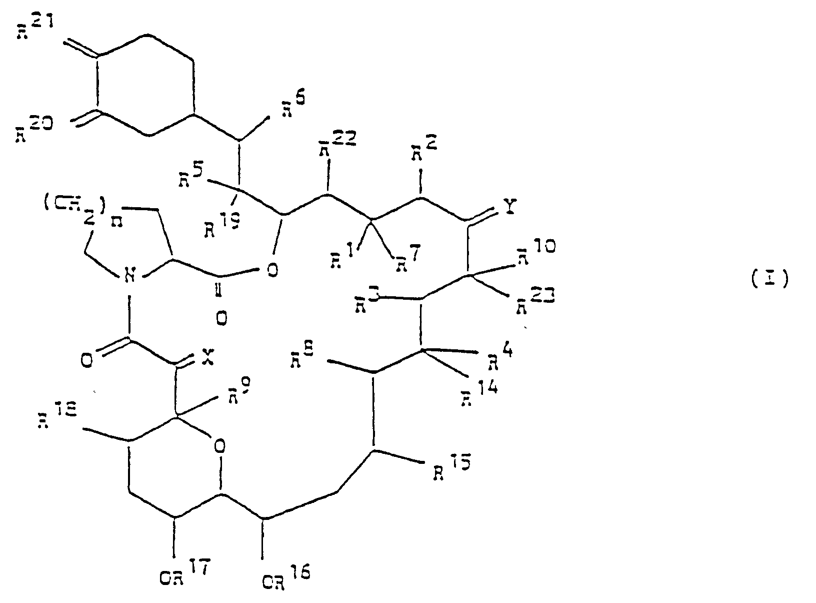 worin jedes vizinale Paar von Substituenten [R¹ und R²], [R³ und R⁴], [R⁵ und R⁶] unabhängig voneinander
a) zwei vizinale Wasserstoffatome darstellt, oder
b) eine zweite Bindung zwischen den vizinalen Kohlenstoffatomen bilden, an die sie gebunden sind;
zusätzlich zu seiner obigen Bedeutung R² eine (C₁-C₆)-Alkylgruppe darstellen kann;
R⁷ H, OH, geschütztes Hydroxy, ausgewählt aus 1-(C₁-C₆)-Alkylthio)(C₁-C₆)alkyloxy, Tri(C₁-C₆)alkylsilyloxy, (C₁-C₆)Alkyl-diphenyl-silyloxy und Acyloxy, oder O-(C₁-C₆)Alkyl darstellt,
R⁸ und R⁹ unabhängig voneinander H oder OH darstellen;
R¹⁰ H, (C₁-C₆)Alkyl, (C₁-C₆)Alkyl, substituiert durch eine oder mehrere Hydroxylgruppen, (C₂-C₆)Alkenyl, (C₂-C₆)-Alkenyl, substituiert durch eine oder mehrere Hydroxylgruppen, oder (C₁-C₆)-Alkyl, substituiert durch =O, darstellt;
X O, (H,OH), (H,H) oder - CH₂O- darstellt;
Y O, (H,OH), (H,H) oder N-NR¹¹R¹² oder N-OR¹³ darstellt;
R¹¹ und R¹² unabhängig voneinander H, (C₁-C₆)Alkyl, Aryl, ausgewählt aus Phenyl, Tolyl, Xylyl, Cumenyl, Mesityl und Naphthyl, oder Tosyl darstellen;
R¹³, R¹⁴, R¹⁵, R¹⁶, R¹⁷, R¹⁸, R¹⁹, R²² und R²³ unabhängig voneinander H oder (C₁-C₆)Alkyl darstellen;
R²⁰ und R²¹ unabhängig voneinander O darstellen, oder abhängig voneinander (R²⁰a, H) beziehungsweise (R²¹a, H) darstellen; R²⁰a und R²¹a unabhängig voneinander OH, O-(C₁-C₆)-Alkyl oder OCH₂OCH₂CH₂OCH₃ darstellen, oder R²¹a geschütztes Hydroxy ist, ausgewählt aus 1-(C₁-C₆)-Alkylthio(C₁-C₆) alkyloxy, Tri(C₁-C₆)alkylsilyloxy, (C₁-C₆)Alkyl-diphenyl-silyloxy und Acyloxy ist,
zusätzlich R²⁰a und R²¹a zusammen ein Sauerstoffatom in einem Epoxidring darstellen können;
n 1 oder 2 ist;
zusätzlich zu ihren obigen Bedeutungen Y, R¹⁰ und R²³ zusammen mit den Kohlenstoffatomen, an die sie gebunden sind, einen heterocyclischen Ring darstellen können, der aus Pyrrolyl und Tetrahydrofuryl ausgewählt ist, und der durch eine oder mehrere Gruppen, ausgewählt aus (C₁-C₆)Alkyl, Hydroxy, (C₁-C₆)Alkyl, substituiert durch eine oder mehrere Hydroxylgruppen, O-(C₁-C₆)Alkyl, Benzyl und -CH₂Se(C₆H₅), substituiert sein können;
oder eines pharmazeutisch verträglichen Slazes davon, und eines pharmazeutisch verträglichen oberflächenaktiven Mittels in einem pharmazeutisch verträglichen nichtwäßrigen Lösungsmittel.

2. Verfahren nach Anspruch 1, worin die Verbindung (I) oder ein pharmazeutisch verträgliches Salz davon und das pharmazeutisch verträgliche oberflächenaktive Mittel in einem Gewichtsverhältnis von 1:1 bis 1:100 vorliegen.

3. Verfahren nach Anspruch 2, worin das pharmazeutisch verträgliche oberflächenaktive Mittel ein Castoröloberflächenaktives Mittel ist.

4. Verfahren nach Anspruch 3, worin das pharmazeutisch verträgliche nichtwäßrige Lösungsmittel Ethylalkohol ist.

5. Verfahren nach Anspruch 4, worin die Verbindung (I) 17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo-[22.3.1.0^{4.9}]octacos-18-en-2,3,10,16- tetraon ist.

## Revendications (Revendications pour l'(les) Etat(s) contractant(s) suivant(s): AT, BE, CH, DE, DK, FR, GB, IT, LI, LU, NL, SE)

1. Solution pharmaceutique comprenant un composé de la formule générale : 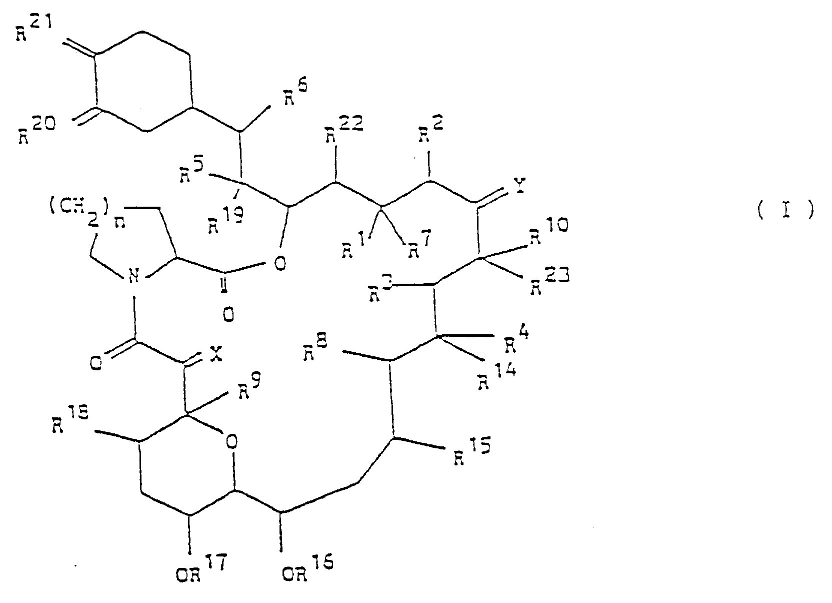 dans laquelle chaque paire adjacente de substituants [R¹ et R²], [R³ et R⁴], [R⁵ et R⁶], indépendamment :
a) représente deux atomes d'hydrogène adjacents, ou
b) forme une seconde liaison entre les atomes de carbone adjacents auxquels elle est attachée;
en plus de sa signification indiquée ci-dessus, R² peut représenter un groupe alkyle en C₁-C₆;
R⁷ représente H, OH, un groupe hydroxy protégé choisi parmi les groupes 1-(alkylthio en C₁-C₆)(alkyloxy en C₁-C₆), tri(alkyle en C₁-C₆)silyloxy, (alkyle en C₁-C₆)-diphényl-silyloxy et acyloxy, ou O-(alkyle en C₁-C₆),
R⁸ et R⁹ représentent indépendamment H ou OH;
R¹⁰ représente H, un groupe alkyle en C₁-C₆, un groupe alkyle en C₁-C₆ substitué par un ou plusieurs groupes hydroxyle, un groupe alcényle en C₂-C₆, un groupe alcényle en C₂-C₆ substitué par un ou plusieurs groupes hydroxyle, ou un groupe alkyle en C₁-C₆ substitué par = O;
X représente O, (H,OH), (H,H) ou - CH₂O-;
Y représente O, (H,OH), (H,H) ou N-NR¹¹R¹² ou N-OR¹³;
R¹¹ et R¹² représentent indépendamment H, un groupe alkyle en C₁-C₆, un groupe aryle choisi parmi les groupes phényle, tolyle, xylyle, cuményle, mésityle et naphtyle ou tosyle;
R¹³, R¹⁴, R¹⁵, R¹⁶, R¹⁷, R¹⁸, R¹⁹, R²² et R²³ représentent indépendamment H ou un groupe alkyle en C₁-C₆;
R²⁰ et R²¹ représentent indépendamment O, ou ils peuvent représenter indépendamment (R²⁰a, H) et (R²¹a, H) respectivement;
R²⁰a et R²¹a représentent indépendamment OH, un groupe O-(alkyle en C₁-C₆) ou OCH₂OCH₂CH₂OCH₃ ou R²¹a est un groupe hydroxy protégé choisi parmi les groupes 1-(alkylthio en C₁-C₆)(alkyloxy en C₁-C₆), tri(alkyle en C₁-C₆)silyloxy, (alkyle en C₁-C₆)diphényl-silyloxy et acyloxy,
de plus, R²⁰a et R²¹a peuvent représenter ensemble un atome d'oxygène dans un cycle époxyde;
n est 1 ou 2;
en plus de leurs significations indiquées ci-dessus, Y, R¹⁰ et R²³, en même temps que les atomes de carbone auxquels ils sont attachés, peuvent représenter un cycle hétérocyclique choisi parmi les groupes pyrrolyle et tétrahydrofuryle, et qui peuvent être substitués par un ou plusieurs groupes choisis parmi les groupes alkyle en C₁-C₆, hydroxy, alkyle en C₁-C₆ substitué par un ou plusieurs groupes hydroxyle, O-(alkyle en C₁-C₆), benzyle et -CH₂Se(C₆H₅),
ou un sel pharmaceutiquement acceptable de celui-ci, un agent tensio-actif pharmaceutiquement acceptable et un solvant non aqueux pharmaceutiquement acceptable.

2. Solution pharmaceutique selon la revendication 1, dans laquelle le composé (I) ou un de ses sels pharmaceutiquement acceptables et l'agent tensio-actif pharmaceutiquement acceptable sont présents dans un rapport pondéral de 1:1 à 1:100.

3. Solution pharmaceutique selon la revendication 2, dans laquelle l'agent tensio-actif pharmaceutiquement acceptable est un agent tensio-actif à l'huile de ricin.

4. Solution pharmaceutique selon la revendication 3, dans laquelle le solvant non aqueux pharmaceutiquement acceptable est l'alcool éthylique.

5. Solution pharmaceutique selon la revendication 4, dans laquelle le composé (I) est le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo-[22.3.1.0^{4.9}]octacos-18-ène-2,3,10,16-tétraone.

6. Procédé pour préparer une solution pharmaceutique, qui est caractérisé en ce qu'on dissout un composé de la formule : 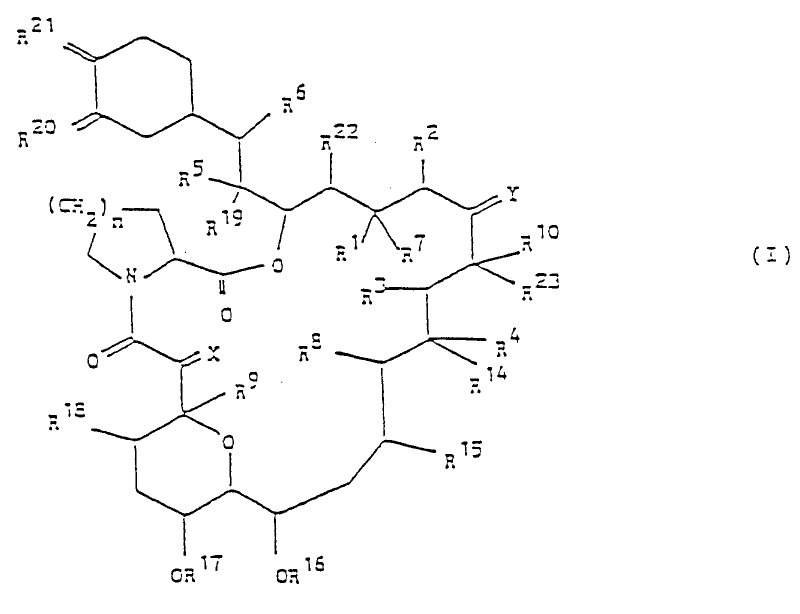 dans laquelle chaque paire adjacente de substituants [R¹ et R²], [R³ et R⁴], [R⁵ et R⁶], indépendamment :
a) représente deux atomes d'hydrogène adjacents, ou
b) forme une seconde liaison entre les atomes de carbone adjacents auxquels elle est attachée;
en plus de sa signification indiquée ci-dessus, R² peut représenter un groupe alkyle en C₁-C₆;
R⁷ représente H, OH, un groupe hydroxy protégé choisi parmi les groupes 1-(alkylthio en C₁-C₆)(alkyloxy en C₁-C₆), tri(alkyle en C₁-C₆)silyloxy, (alkyle en C₁-C₆)-diphényl-silyloxy et acyloxy, ou O-(alkyle en C₁-C₆),
R⁸ et R⁹ représentent indépendamment H ou OH;
R¹⁰ représente H, un groupe alkyle en C₁-C₆, un groupe alkyle en C₁-C₆ substitué par un ou plusieurs groupes hydroxyle, un groupe alcényle en C₂-C₆, un groupe alcényle en C₂-C₆ substitué par un ou plusieurs groupes hydroxyle, ou un groupe alkyle en C₁-C₆ substitué par = O;
X représente O, (H,OH), (H,H) ou - CH₂O-;
Y représente O, (H,OH), (H,H) ou N-NR¹¹R¹² ou N-OR¹³;
R¹¹ et R¹² représentent indépendamment H, un groupe alkyle en C₁-C₆, un groupe aryle choisi parmi les groupes phényle, tolyle, xylyle, cuményle, mésityle et naphtyle ou tosyle;
R¹³, R¹⁴, R¹⁵, R¹⁶, R¹⁷, R¹⁸, R¹⁹, R²² et R²³ représentent indépendamment H ou un groupe alkyle en C₁-C₆;
R²⁰ et R²¹ représentent indépendamment O, ou ils peuvent représenter indépendamment (R²⁰a, H) et (R²¹a, H) respectivement;
R²⁰a et R²¹a représentent indépendamment OH, un groupe O-(alkyle en C₁-C₆) ou OCH₂OCH₂CH₂OCH₃ ou R²¹a est un groupe hydroxy protégé choisi parmi les groupes 1-(alkylthio en C₁-C₆)(alkyloxy en C₁-C₆), tri(alkyle en C₁-C₆)silyloxy, (alkyle en C₁-C₆)diphényl-silyloxy et acyloxy,
de plus, R²⁰a et R²¹a peuvent représenter ensemble un atome d'oxygène dans un cycle époxyde;
n est 1 ou 2;
en plus de leurs significations données ci-dessus, Y, R¹⁰ et R²³, en même temps que les atomes de carbone auxquels ils sont attachés, peuvent représenter un cycle hétérocyclique choisi parmi les groupes pyrrolyle et tétrahydrofuryle, et qui peuvent être substitués par un ou plusieurs groupes choisis parmi les groupes alkyle en C₁-C₆, hydroxy, alkyle en C₁-C₆ substitué par un ou plusieurs groupes hydroxyle, O-(alkyle en C₁-C₆), benzyle et -CH₂Se(C₆H₅),
ou un de ses sels pharmaceutiquement acceptables, et un agent tensio-actif pharmaceutiquement acceptable dans un solvant non aqueux pharmaceutiquement acceptable.

## Revendications (Revendications pour l'(les) Etat(s) contractant(s) suivant(s): ES, GR)

1. Procédé pour préparer une solution pharmaceutique, qui est caractérisé en ce qu'on dissout un composé de la formule : 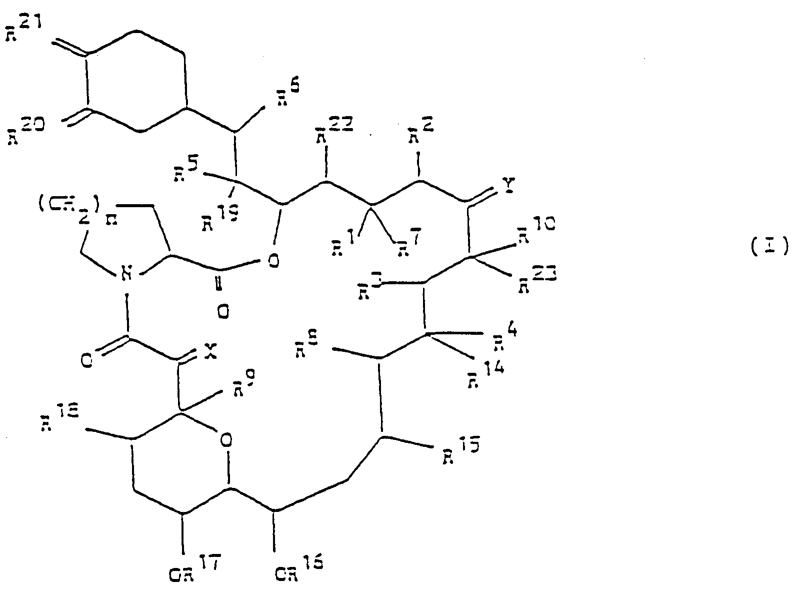 dans laquelle chaque paire adjacente de substituants [R¹ et R²], [R³ et R⁴], [R⁵ et R⁶], indépendamment :
a) représente deux atomes d'hydrogène adjacents, ou
b) forme une seconde liaison entre les atomes de carbone adjacents auxquels elle est attachée;
en plus de sa signification indiquée ci-dessus, R² peut représenter un groupe alkyle en C₁-C₆;
R⁷ représente H, OH, un groupe hydroxy protégé choisi parmi les groupes 1-(alkylthio en C₁-C₆) (alkyloxy en C₁-C₆), tri(alkyle en C₁-C₆)silyloxy, (alkyle en C₁-C₆)-diphényl-silyloxy et acyloxy, ou O-(alkyle en C₁-C₆),
R⁸ et R⁹ représentent indépendamment H ou OH;
R¹⁰ représente H, un groupe alkyle en C₁-C₆, un groupe alkyle en C₁-C₆ substitué par un ou plusieurs groupes hydroxyle, un groupe alcényle en C₂-C₆, un groupe alcényle en C₂-C₆ substitué par un ou plusieurs groupes hydroxyle, ou un groupe alkyle en C₁-C₆ substitué par = O;
X représente O, (H,OH), (H,H) ou - CH₂O-;
Y représente O, (H,OH), (H,H) ou N-NR¹¹R¹² ou N-OR¹³;
R¹¹ et R¹² représentent indépendamment H, un groupe alkyle en C₁-C₆, un groupe aryle choisi parmi les groupes phényle, tolyle, xylyle, cuményle, mésityle et naphtyle ou tosyle;
R¹³, R¹⁴, R¹⁵, R¹⁶, R¹⁷, R¹⁸, R¹⁹, R²² et R²³ représentent indépendamment H ou un groupe alkyle en C₁-C₆;
R²⁰ et R²¹ représentent indépendamment O, ou ils peuvent représenter indépendamment (R²⁰a, H) et (R²¹a, H) respectivement;
R²⁰a et R²¹a représentent indépendamment OH, un groupe O-(alkyle en C₁-C₆) ou OCH₂OCH₂CH₂OCH₃ ou R²¹a est un groupe hydroxy protégé choisi parmi les groupes 1-(alkylthio en C₁-C₆) (alkyloxy en C₁-C₆), tri(alkyle en C₁-C₆)silyloxy, (alkyle en C₁-C₆)diphényl-silyloxy et acyloxy,
de plus, R²⁰a et R²¹a peuvent représenter ensemble un atome d'oxygène dans un cycle époxyde;
n est 1 ou 2;
en plus de leurs significations indiquées ci-dessus, Y, R¹⁰ et R²³, en même temps que les atomes de carbone auxquels ils sont attachés, peuvent représenter un cycle hétérocyclique choisi parmi les groupes pyrrolyle et tétrahydrofuryle, et qui peuvent être substitués par un ou plusieurs groupes choisis parmi les groupes alkyle en C₁-C₆, hydroxy, alkyle en C₁-C₆ substitué par un ou plusieurs groupes hydroxyle, O-(alkyle en C₁-C₆), benzyle et -CH₂Se(C₆H₅),
ou un de ses sels pharmaceutiquement acceptables, et un agent tensio-actif pharmaceutiquement acceptable dans un solvant non aqueux pharmaceutiquement acceptable.

2. Procédé selon la revendication 1, dans lequel le composé (I) ou un de ses sels pharmaceutiquement acceptables et l'agent tensio-actif pharmaceutiquement acceptable sont présents dans le rapport pondéral de 1:1 à 1:100.

3. Procédé selon la revendication 2, dans lequel l'agent tensio-actif pharmaceutiquement acceptable est un agent tensio-actif à l'huile de ricin.

4. Procédé selon la revendication 3, dans lequel le solvant non aqueux pharmaceutiquement acceptable est l'alcool éthylique.

5. Procédé selon la revendication 4, dans lequel le composé (I) est le 17-allyl-1,14-dihydroxy-12-[2-(4-hydroxy-3-méthoxcyclohexyl)-1-méthylvinyl]-23,25-diméthoxy-13,19,21,27-tétraméthyl-11,28-dioxa-4-azatricyclo-[22.3.1.0^{4.9}]octacos-18-ène-2,3,10,16-tétraone.
